# FASCICULE DE BREVET EUROPEEN

(11) **EP 1 225 630 B1**
(45) Date de publication et mention de la délivrance du brevet: **22.04.2009**
(21) Numéro de dépôt: 02290136.7
(22) Date de dépôt: 18.01.2002
(51) Int. Cl.: H01L 23/31, A61N 1/375, H01L 25/065, H01L 21/56

(54) **Procédé de fabrication de circuits électroniques hybrides pour dispositifs médicaux implantables actifs**
Verfahren zur Herstellung von elektronischen Hybridschaltungen für aktive medizinische implantierbare Vorrichtungen
Method for making hybrid electronic circuits for active implantable medical devices

(30) Priorité: 19.01.2001 FR 0100726
(43) Date de publication de la demande: 24.07.2002
(73) Titulaire: ELA MEDICAL (Société anonyme), 92541 Montrouge (FR)
(72) Inventeur: van Campenhout, Yves, 94190 Villeneuve Saint Georges (FR); Gilet, Dominique, 92160 Antony (FR); Legay, Thierry, 91640 Fontenay les Bris (FR)
(74) Mandataire: Dupuis-Latour, Dominique

(56) Documents cités:
- EP-A- 0 923 130
- CH-A- 689 217
- US-A- 3 690 325
- US-A- 5 782 891
- US-A- 5 784 264
- US-A- 5 954 751
- US-A- 5 963 429

## Description

L'invention concerne la technologie de fabrication des circuits électroniques hybrides des dispositifs médicaux implantables actifs.

Elle sera principalement décrite dans le cas d'un stimulateur cardiaque, mais il ne s'agit là que d'un exemple de mise en oeuvre de l'invention, qui est applicable de façon beaucoup plus générale à une très grande variété de "dispositifs médicaux implantables actifs" tels que définis par la Directive 90/385/CE du 20 juin 1990 du Conseil des communautés européennes, définition qui inclut, outre les stimulateurs cardiaques, les défibrillateurs et/ou cardioverteurs, les appareils neurologiques, les pompes de diffusion de substances médicales, les implants cochléaires, les capteurs biologiques implantés, etc.

Ces dispositifs médicaux implantables actifs (ci-après "dispositifs" ou "dispositifs implantables") se présentent sous la forme d'un boîtier contenant une pile d'alimentation et une plaquette de circuit hybride (ci-après "circuit électronique") supportant et interconnectant l'ensemble des composants, actifs et passifs permettant le recueil et l'analyse des signaux, la génération des impulsions électriques, la mémorisation de diverses informations de suivi médical, le pilotage des diverses fonctions du dispositif, etc. Une tête de connecteur reliée à ce circuit électronique assure la liaison électrique et mécanique à des éléments externes, notamment des sondes de recueil de signaux et/ou d'application d'impulsions.

Comme on le comprend aisément, ces dispositifs implantés requièrent une miniaturisation poussée du circuit électronique, compte tenu de la place réduite disponible à l'intérieur du boîtier.

Plus précisément, outre divers composants actifs et passifs généralement reportés en technologie CMS, ainsi que des éléments de connectique, le circuit électronique porte une ou plusieurs puces réalisées par microphotolithographie d'une plaquette de semiconducteur, découpée en puces individuelles.

Ces puces, ainsi que les autres composants actifs et passifs, sont reportés sur un substrat individuel unique pour constituer le circuit électronique du dispositif.

Plusieurs techniques sont aujourd'hui employées pour réaliser ces circuits électroniques, mais toutes présentent des inconvénients qui leur sont propres.

Une première technique consiste à utiliser un *"chip carrier",* qui est un petit boîtier individuel au fond duquel la puce est collée, puis câblée par des microfils ("*wire bonding*") ; le *chip carrier* est finalement scellé par un capot métallique, puis reporté sur le substrat du circuit hybride. Cette méthode est coûteuse, car elle nécessite un traitement individuel de chaque puce, et requiert autant de *chip carriers* que de puces ; elle conduit également à une surface occupée importante, le *chip carrier* étant nécessairement plus grand que la puce qu'il incorpore.

Une autre technique, décrite par exemple dans le EP-A-0 923 130, est celle dite du "montage en cavité commune". Le substrat du circuit hybride n'est pas plan, mais comporte déjà une cavité, suffisamment grande pour accueillir toutes les puces, qui sont collées et câblées par une technique de prise de connexion directe par le dessus de la puce, telle que celle décrite dans le FR-A-2 691 836.

Un capot commun ou une résine coulée dans la cavité couvre ensuite la totalité de la cavité. Ce procédé présente l'avantage de traiter globalement toutes les puces d'un même circuit hybride, mais il est délicat à mettre en oeuvre (creusement ou formation de la cavité, prise de connexion par le dessus de la puce), avec corrélativement un prix de revient élevé. De plus, du point de vue de la surface occupée, les murs de la cavité occupent une surface perdue non négligeable.

Une autre technique dite du montage "*chip-on-board*" consiste à coller les puces sur le substrat-hôte puis, après câblage, les recouvrir individuellement d'une goutte ou "globe" de résine protectrice (voir notamment le US-A-4 784 872). La puce ainsi enrobée par un *"glob-top"* occupe une place relativement importante sur le substrat, car il est nécessaire de prévoir une marge suffisante pour l'écoulement naturel de la goutte de résine d'enrobage. De plus, le coût de la technique est relativement élevé, car il est nécessaire de traiter individuellement chaque puce de chaque circuit. Enfin, l'état mécanique de la surface qui contient les puces en *glob-top* n'est pas bien contrôlé et ne permet pas une insertion ajustée dans un dispositif implantable, où chaque millimètre cube est compté.

Le WO-A-99/41786 décrit une technique d'enrobage de sous-ensembles d'un circuit de stimulateur cardiaque, où les sous-ensembles sont recouverts collectivement, avant d'être découpés, d'une couche de résine d'enrobage. Toutefois, ce document n'enseigne que l'enrobage de sous-en-sembles, qui doivent être ensuite reportés sur un substrat commun formant le substrat du circuit électronique proprement dit de l'implant. De plus, cette technique ne permet pas d'implanter des composants sur les deux faces, puisque l'une des faces du sous-ensemble est réservée au report sur le substrat du circuit électronique proprement dit.

La présente invention propose une nouvelle technique de fabrication d'un circuit électronique de dispositif médical implantable actif palliant l'ensemble des inconvénients cités plus haut des procédés connus, ainsi qu'un circuit électronique réalisé selon ce procédé.

Le procédé de l'invention est exposé dans la revendication 1. Les sous-revendications 2 et 3 visent des formes de mise en oeuvre avantageuses.

L'invention vise également, en tant que produit nouveau, un circuit électronique de dispositif médical implantable actif réalisé par le procédé précité, comprenant les caractéristiques énoncées à la revendication 4.

On va maintenant décrire un exemple de mise en oeuvre de l'invention, en référence aux dessins annexés.
Les figures 1 et 2 illustrent une plaque-substrat collective sur laquelle ont été reportées et câblées les puces de plusieurs circuits de dispositifs implantables, respectivement avant et après coulage de la résine d'enrobage.
Les figures 3 et 4 sont des vues en perspective, respectivement de dessous et de dessus, du circuit électronique fini, réalisé selon le procédé de l'invention.

Essentiellement, la présente invention propose de réaliser simultanément plusieurs circuits électroniques de dispositif implantable sur une même plaque-substrat collective, d'enrober par une résine, en une seule et même étape, la totalité de la surface de cette plaque-support, puis d'individualiser chacun des circuits par découpe de la plaque-substrat collective enrobée pour former les divers circuits électroniques individuels, la seconde face du substrat (celle n'ayant pas reçu de résine d'enrobage) étant pourvue de métallisations pour le report de composants CMS.

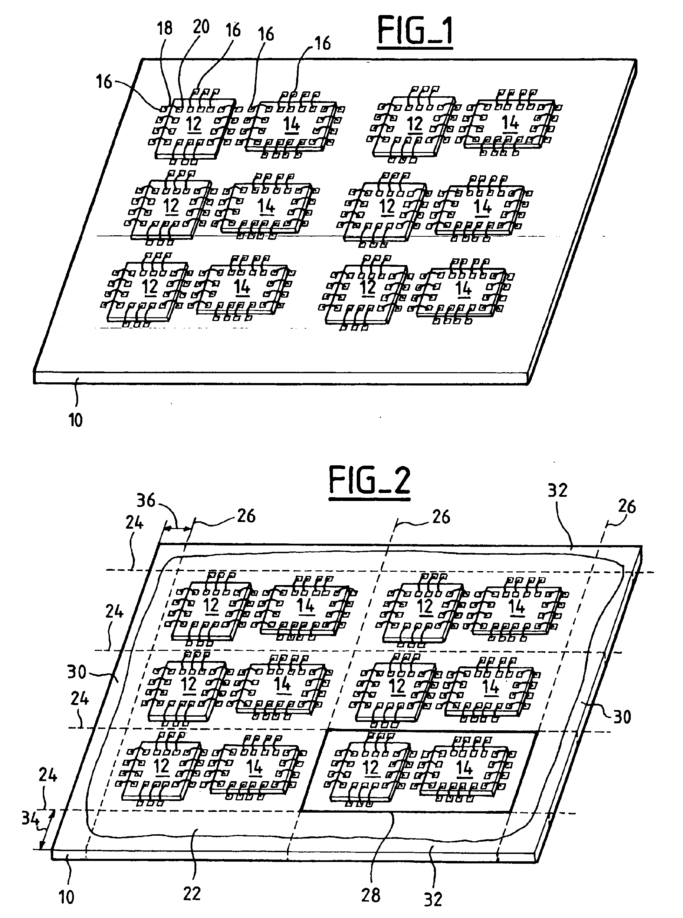

Sur la figure 1, on a représenté une telle plaque-support collective, plane, référencée 10, qui peut être réalisée en un matériau en lui-même connu tel que céramique (plusieurs couches cocuites), époxy rigide, etc. le matériau étant choisi selon les propriétés mécaniques et électriques que l'on souhaite pour le circuit électronique. La plaque-substrat incorpore les divers conducteurs d'interconnexion des puces et autres composants qu'elle est destinée à recevoir.

Dans l'exemple illustré, chaque circuit électronique comporte deux puces 12, 14 obtenues chacune par microphotolithographie et découpe d'une plaquette de semi-conducteur. Ces puces 12, 14 sont ensuite reportées et collées sur la plaque-substrat collective 10 lors d'une première étape du procédé. Sur la figure 1, on a ainsi illustré douze puces, correspondant à six circuits électroniques individuels de dispositif implantable, toutes reportées et collées sur la plaque-substrat 10.

Une machine de *wire bonding* assure ensuite le câblage des différentes puces, par mise en place de minces fils de liaison 18 reliant des plots de contact 16 formés sur la plaque-substrat 10 à des plots homologues 20 de chacune des puces.

L'étape suivante, illustrée figure 2, consiste à couler une résine d'enrobage (ou "d'encapsulation") 22 sur l'ensemble de la plaque-substrat 10, de manière à recouvrir toutes les puces et leurs liaisons électriques. La résine est choisie en fonction des caractéristiques de résistance mécanique attendues pour le circuit électronique ; il s'agit généralement d'un matériau polymérisable tel qu'un polyimide, un polymère époxy, une résine silicone, etc. susceptible d'être coulée localement et durcie *in situ*, par exemple par exposition aux UV ou passage en température. De préférence, après polymérisation, le matériau durci, bien que rigide, présente néanmoins une souplesse suffisante pour ne générer qu'une contrainte minimale, insusceptible de voiler la plaque-substrat 10.

La résine d'enrobage que l'on vient de couler est de préférence lissée par une raclette. De façon naturelle, la résine va prendre un profil plan (à l'exception des bords) par combinaison des forces de gravité et de tension superficielle.

Sur la figure 2, les références 24 et 26 sont des lignes de repérage délimitant l'étendue de chacun des circuits électroniques individuels (la référence 28 en trait plein illustre le contour d'un tel circuit électronique, considéré isolément avec ses deux puces 12, 14). Par rapport aux dimensions des circuits électroniques, les dimensions de la plaque-substrat 10 sont choisies de manière à conserver des marges 30, 32 périphériques suffisantes pour que le bord de la masse de résine 22 atteigne les régions correspondantes 34, 36 de ces marges périphériques, en offrant une surface pratiquement plane sur la totalité de l'étendue des divers circuits électroniques individuels.

Après durcissement de la résine, la plaque-substrat et sa couche de résine d'enrobage sont découpées selon les lignes 24 et 26, de façon à individualiser chacun des circuits électroniques.

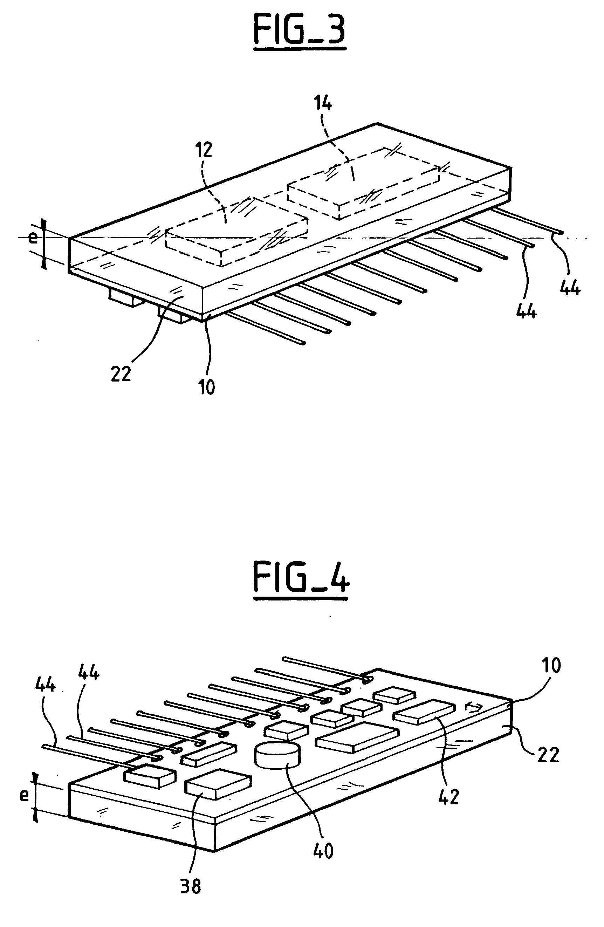

Comme on peut le voir sur les figures 3 et 4, chacun de ces circuits individuels présente une face libre (le recto de la plaque-substrat illustré figure 2) comportant des métallisations pour le report de composants CMS actifs ou passifs tels que 38, 40, 42 et de broches de liaison 44 à la connectique de l'implant médical (figure 4).

Sur la face opposée (figure 3), le circuit présente simplement une couche 22 de résine, plane et rigide, d'épaisseur e (typiquement 0,8 mm environ), qui enrobe complètement les puces 12 et 14.

En complément, après durcissement de la résine et avant découpe de la plaque-substrat 10, il est possible de prévoir le dépôt d'une couche métallique sur l'ensemble de la surface de la résine. Une telle couche métallique peut assurer plusieurs fonctions, notamment :
- blindage électrique (en reliant cette couche métallique à la masse),
- écran à la pénétration de l'humidité et des contaminants chimiques,
- protection mécanique accrue.

Le procédé que l'on vient de décrire présente de nombreux avantages, en particulier :
- bonne densité d'occupation de la surface du circuit électronique individuel, sans perte d'une marge pour l'écoulement de la résine comme dans le cas du procédé *glob-top* ; en effet, dans l'invention, la marge n'est réservée qu'à la périphérie de la plaque-substrat collective, et n'a pas d'incidence sur les dimensions individuelles de chacun des circuits électroniques.
- optimisation des coûts, car les opérations de collage, de *wire bonding* et de coulage de la résine sont communes à plusieurs circuits : si l'on réalise ainsi sur la plaque-substrat collective *n* circuits, on diminue d'un facteur *n* le nombre d'entrées-sorties du process de fabrication.
- bonne planéité de surface de la résine (qui s'est étalée simplement par gravité) et, du fait du sciage, flancs droits du circuit, offrant donc un bon référentiel mécanique, pour un positionnement et un ajustage précis et aisés.
- bonne protection des puces vis-à-vis du milieu extérieur.

## Revendications

1. Un procédé de fabrication de circuits électroniques hybrides pour dispositifs médicaux implantables actifs, comprenant les étapes suivantes :
a) préparation d'une plaque-substrat collective (10) comportant, à son recto, des plots de contact (16) pour des puces (12, 14) et, à son verso, des métallisations pour des composants CMS actifs ou passifs (38, 40, 42) et/ou des éléments de connectique (44), cette plaque-substrat collective comportant une répétition des mêmes motifs de plots et de métallisations pour une pluralité correspondante de circuits électroniques de dispositifs implantables,
b) collage au recto de la plaque-substrat collective (10) d'une pluralité de puces (12, 14) avec, pour chaque circuit électrique de dispositif implantable, une ou plusieurs puces correspondantes,
c) câblage des puces (12, 14) aux plots de contact (16) qui leur sont associés,
d) coulage, sur la surface au recto de la plaque-substrat collective, d'une couche de résine d'enrobage (22),
e) durcissement de la résine d'enrobage,
f) sciage de la plaque-substrat collective, pour individualiser une pluralité de substrats pourvus chacun à leur recto d'une ou plusieurs puces enrobées par la résine, chacun de ces substrats individuels correspondant à un circuit électrique individuel de dispositif implantable,
g) report des composants CMS (38, 40, 42) et/ou des éléments de connectique (44) au verso de chacun des substrats individuels, de manière à constituer les circuits électriques complets des dispositifs implantables, procédé **caractérisé en ce que** :
- ladite plaque-substrat collective (10) est une plaque-substrat plane,
- le câblage des puces est une étape avec mise en place de fils de liaison (18) reliant lesdits plots de contact (16) de la plaque-substrat plane (10) à des plots homologues (20) de chacune des puces, et
- la couche de résine est une couche d'épaisseur uniforme recouvrant toutes les puces et leurs liaisons électroniques.

2. Le procédé de la revendication 1, dans lequel la plaque-substrat collective comporte une région périphérique marginale perdue (30, 32) et dans lequel, à l'étape d), la résine s'étale librement par gravité jusque sur cette région périphérique et, à l'étape f), cette région est éliminée au sciage.

3. Le procédé de la revendication 1, dans lequel il est prévu, après l'étape e), une étape de dépôt d'une couche métallique uniforme sur la surface libre de la couche de résine d'enrobage durcie.

4. Un circuit électronique de dispositif médical implantable actif réalisé par le procédé des revendications 1 à 3, ce circuit étant formé d'un substrat supportant, à son recto, une ou plusieurs puces (12, 14) et, à son verso, des composants CMS actifs ou passifs (38, 40, 42) et/ou des éléments de connectique (44),
**caractérisé en ce que** le substrat est un substrat plan et **en ce que** le circuit comporte en outre, au recto du substrat (10), une couche de résine d'enrobage noyant les circuits et puces et leurs liaisons électriques, cette couche étant d'épaisseur uniforme et s'étendant d'un bord à l'autre du substrat dans les deux dimensions de celui-ci, les flancs du substrat et de sa couche de résine étant des flancs plans perpendiculaires à la surface du substrat.

## Claims

1. A method for manufacturing hybrid electronic circuits for active implantable medical devices, comprising
a) preparing a collective substrate plate (10) carrying on the front side bump contacts (16) for chips (12, 14) and on the back side metallization areas for active or passive SMC components (38, 40, 42) and/or connecting elements (44), said collective substrate plate comprising a repetition of the same patterns of bumps and of metallization areas for a corresponding plurality of electronic circuits for implantable devices,
b) bonding on the front side of the collective substrate plate (10) a plurality of chips (12, 14), each electronic circuit for implantable device having one or more corresponding chips,
c) wiring chips (12, 14) to the bump contacts (16) that are associated to them,
d) pouring an embedding resin layer (22) onto the collective substrate plate front side surface,
e) hardening the embedding resin,
f) sawing the collective substrate plate in order to individualize a plurality of substrates the front side of each of them being provided with one or more chips embedded in the resin, each of these individual substrates corresponding to an electronic circuit for implantable devices,
g) mounting the SMC components (38, 40, 42) and/or of the connecting elements (44) onto the back side of each of the individual substrates, so as to complete the electronic circuits of the implantable devices,
**characterized in that** :
- said collective substrate plate (10) is a flat substrate-plate,
- wiring of the chips is a step with putting in place of jumper wires (18) linking said bump contacts (16) of the flat substrate plate (10) to homologous bumps (20) of each of the chips, and
- the resin layer is a layer having a uniform thickness covering all the chips and their electrical links.

2. The method of claim 1, wherein the collective substrate-plate comprises a lost marginal peripheric region (30, 32) and wherein in the step d) the resign flows out freely by gravity until arriving on said peripheric region, and in the step f) said region is eliminated during sawing.

3. The method of claim 1, wherein after the step e) a step of depositing a uniform metallic layer onto the free surface of the hardened embedding resin layer.

4. An electronic circuit for an active implantable medical device produced by the method of claims 1 to 3, said circuit consisting of a substrate carrying on its front side one or more chips (12, 14) and on its back side active or passive SMC components (38, 40, 42) and/or connecting elements (44),
**characterized in that** the substrate is a flat substrate and **in that** the circuit further comprises an embedding resin layer embedding the circuit, the chips and their electrical links, said layer having a uniform thickness and extending from one edge to the other of the substrate in both dimensions of the latter, wherein the flanks of the substrate and of its resin layer are flat flanks perpendicular to the surface of the substrate.

## Patentansprüche

1. Ein Verfahren zum Herstellen elektronischer Hybridschaltungen für aktive, implantierbare medizinische Vorrichtungen, wobei das Verfahren folgende Schritte enthält :
a) Vorbereitung einer kollektiven Substratplatte (10) umfassend auf ihrer. Vorderseite Bump-kontakte (16) für Chips (12, 14) und auf der Rückseite Metallisierungen für aktive oder passive SMT-Bauelemente (38, 40, 42) und/oder Anschlusselemente (44), wobei diese kollektive Substratplatte eine Wiederholung der selben Muster von Bump-kontakten und Metallisierungen für eine Vielzahl von elektronischen Stromkreisen für implantierbare Vorrichtungen aufweist,
b) Verkleben auf der Vorderseite der kollektiven Substratplatte (10) einer Vielzahl von Chips (12, 14) mit einem oder vielen für jeden Stromkreis der implantierbaren Vorrichtung bestimmten, entsprechenden Chips,
c) Verdrahtung der Chips (12, 14) zu den ihnen zugeordneten Bump-kontakten,
d) Giessen einer Schicht eines Einbettharzes (22) auf die Vorderseitenoberfäche der kollektiven Substratplatte,
e) Härtung des Einbettharzes,
f) Sägen der kollektiven Substratplatte zur Individualisierung einer Vielzahl von Substraten, wobei jede von letzteren auf ihrer Vorderseite mit einer oder mehreren im Harz eingebetteten Chips versehen ist, wobei jedes jener Substrate einen individuellen Stromkreis für eine implantierbare Vorrichtung entspricht,
g) Übertragen der zum Oberflächenmontage geeigneten Bauelemente (38, 40, 42) und/oder der Anschlusselemente (44) auf die Rückseite jeder der individuellen Substrate, um vollständige Stromkreise der implantierbaren Vorrichtungen zu bilden,
wobei das Verfahren **dadurch gekennzeichnet ist, dass**
- die besagte kollektive Substratplatte (10) eine flache Substratplatte ist,
- die Verdrahtung der Chips ein Schritt mit Platzierung von Überbrückungsdrähten (18) zwischen besagten Bump-kontakten (16) der Substratplatte (10) und homologen Bump-kontakten (2) jeder der Chips ist,
- die Harzschicht eine gleichmässige Dicke aufweisende, alle Chips und ihre elektrischen Verbindungen abdeckende Schicht ist.

2. Das Verfahren nach Anspruch 1, in welchem die kollektive Substratplatte einen verlorenen, peripherischen Randbereich (30, 32) aufweist und in welchem im Schritt d) das Harz bis diesem peripherischen Bereich frei verläuft und im Schritt f) dieser Bereich abgesägt wird.

3. Das Verfahren nach Anspruch 1, in welchem nach dem Schritt e) ein Schritt zum Auftragen einer gleichmässigen metallischen Schicht auf die freie Oberfläche der gehärteten Einbettharzschicht vorgesehen ist.

4. Ein nach dem Verfahren der Ansprüche 1 bis 3 hergestellter elektronischer Stromkreis einer implantierbaren medizinischen Vorrichtung, wobei dieser Stromkreis aus einem auf seiner Vorderseite ein oder mehrere Chips (12, 14) und auf seiner Rückseite aktive oder passive SMT-Bauelemente (38, 40, 42) und/oder Anschlusselemente (44) tragenden Substrat besteht, **dadurch gekennzeichnet, dass** das Substrat ein flaches Substrat ist und dass der Stromkreis ausserdem auf der Vorderseite des Substrats eine die Stromkreise und Chips einbettende Einbettharzschicht besitzt, wobei diese Schicht eine gleichmässige Dicke hat und sich von einem Rand des Substrats zum anderen in beiden Dimensionen desselben erstreckt, wobei die Flanken des Substrats und seiner Harzschicht flache, zur Oberfläche des Substrats senkrechte Flanken sind.
